# Europäisches Patentamt
# European Patent Office
# Office européen des brevets

(19)

(11) Veröffentlichungsnummer: **0 167 745**
**B1**

(12) **EUROPÄISCHE PATENTSCHRIFT**

(45) Veröffentlichungstag der Patentschrift:
14.10.87

(51) Int. Cl.⁴: **A 61 M 1/14**

(21) Anmeldenummer: **85105660.6**

(22) Anmeldetag: **08.05.85**

(54) **Hämodialysevorrichtung.**

(30) Priorität: **08.05.84 DE 3416955**

(43) Veröffentlichungstag der Anmeldung:
**15.01.86 Patentblatt 86/3**

(45) Bekanntmachung des Hinweises auf die Patenterteilung:
**14.10.87 Patentblatt 87/42**

(84) Benannte Vertragsstaaten:
**DE FR GB IT**

(56) Entgegenhaltungen:
**AT-B-322 727
DE-A-2 328 593
DE-A-2 524 978
DE-A-2 548 759
DE-A-2 651 810
DE-A-2 926 681
DE-A-3 202 831
DE-B-2 200 481**

(73) Patentinhaber: **Fresenius AG, Gluckensteinweg 5,
D-6380 Bad Homburg (DE)**

(72) Erfinder: **Polaschegg, Hans- Dietrich, Dr.,
Grünwiesenweg 9, D-6370 Oberursel 4 (DE)**

(74) Vertreter: **Luderschmidt, Wolfgang, Dr. Dipl-
Chem., Görtz, Dr. Fuchs, Dr. Luderschmidt
Patentanwälte Sonnenberger Strasse 100
Postfach 26 26, D-6200 Wiesbaden (DE)**

EP 0 167 745 B1

## Beschreibung

Die Erfindung betrifft eine Hämodialysevorrichtung mit einem Dialysator, der zwei durch eine Membran getrennte Kammern aufweist, von denen die eine in einen Dialysierflüssigkeitsweg und die andere in einen Blutweg geschaltet ist, mit einer Dialysierflüssigkeitsquelle, mit einer im Dialysierflüssigkeitsweg angeordneten, den Transmembrandruck regelnden Saugpumpe, mit einer stromauf des Dialysators im Dialysierflüssigkeitsweg angeordneten Drosseleinrichtung, mit einem stromab der Saugpumpe in den Dialysierflüssigkeitsweg eingeschalteten Umschaltventil, mit einer von dem Umschaltventil abgehenden Leitung, die zum Dialysierflüssigkeitsweg stromauf des Dialysators zurückgeführt ist, mit einer mit dem Dialysierflüssigkeitsweg verbundenen Volumenmeßeinrichtung zur Messung des Volumens der ultrafiltrierten Flüssigkeitsmenge, mit einer Einrichtung zur Bestimmung des Transmembrandrucks im Dialysator und mit einer Regeleinrichtung, die mit der Einrichtung zur Bestimmung des Transmembrandrucks verbunden ist und die Saugpumpe regelt.

Die eingangs erwähnte Hämodialysevorrichtung oder künstliche Niere soll harnpflichtige Substanzen entfernen und den Flüssigkeits-, Elektrolyt- und Säurebasen-Haushalt regulieren.

Wenngleich Ansätze vorhanden sind, diese Regulierungen aufgrund objektiver, physiologischer und kontinuierlich zu messender Parameter vorzunehmen, beispielsweise durch die kontinuierliche Messung des Hämotokrit im extrakorporalen Kreislauf, so wird heute noch ausschließlich auf der Basis von empirisch festgestellten Ideal- oder Sollwerten dialysiert (vgl. ASAIO 7 (1983), S. 410). Das Gros der heute in Verwendung befindlichen Geräte erlaubt dabei noch nicht einmal die präzise Einhaltung der vom Arzt vorgegebenen Parameter, geschweige denn eine gezielte Steuerung dieser Geräte.

Ein Bereich, in dem gewisse Fortschritte erzielt worden sind, ist der Bereich der Ultrafiltrationskontrolle, bei dem im zunehmenden Maß sog. volumenkontrollierte Dialysegeräte eingesetzt werden, die eine genaue Ultrafiltration erlauben, sofern bestimmte apparatetechnische Voraussetzungen gegeben sind. Bei solchen Vorrichtungen wird die Ultrafiltration in einem geschlossenen Volumen Dialysierflüssigkeit vorgenommen, die periodisch ausgewechselt wird. Nur aus einem solchen geschlossenen System, das in dem Dialysegerät A 2008 C der Anmelderin verwirklicht ist, ist gewährleistet, daß eine genaue Ultrafiltrationskontrolle durchgeführt werden kann.

Andererseits befinden sich noch in einer Vielzahl von Ländern einfache Single-Pass-Geräte aus Kostengründen im Einsatz, die derzeit nicht ausgewechselt werden können, obwohl die Vorteile volumengesteuerter Vorrichtungen für den Patienten hinreichend nachgewiesen und anerkannt sind.

Infolgedessen mußten Einrichtungen entwickelt werden, mit denen die bekannten einfachen Hämodialysevorrichtungen überprüft und eingestellt werden können.

Der Transfer von Flüssigkeit vom Blutkreislauf zum Dialysierflüssigkeitskreislauf, d.h. die ultrafiltrierte Flüssigkeit, läßt sich durch Einrichtungen sowohl auf der Blut- als auch auf der Dialysierflüssigkeitsseite verfolgen. Folgende Verfahren sind hierzu bekannt:

Wie bereits vorstehend erwähnt, läßt sich eine Volumenkontrolle durch eine Bilanziervorrichtung sicherstellen, die sicherstellt, daß die zum und vom Dialysator durchströmenden Flüssigkeitsmengen gleich groß sind. Die zu ultrafiltrierende Menge wird dieser Bilanziervorrichtung mit einer zusätzlichen Einrichtung, nämlich einer Ultrafiltratpumpe, entnommen, so daß letztlich das Ultrafiltrat exakt bestimmt werden kann. Eine derartige Vorrichtung ist beispielsweise aus der DE-A-28 38 414 bekannt.

Weiterhin kann eine Relativdurchflußmessung der zufliessenden und abfließenden Flüssigkeitsmengen, also der Mengen von Blut oder insbesondere Dialysierflüssigkeit, vorgenommen werden. Durch Differenzbildung/Zeiteinheit, Vergleich mit einer vorgegebenen Sollrate und Steuerung des Transmembrandrucks kann die gewünschte Sollrate erreicht werden. Dieses Prinzip ist beispielsweise auf der Dialysierflüssigkeitsseite bei dem Ultrafiltrationsmodul UFM-2 der Firma Gambro verwirklicht. Für die Blutseite ist eine solche Vorrichtung in der DE-A-33 13 421 (= EP-A-0 122 604, veröffentlicht am 24.10.84) angegeben.

Ein weiteres Verfahren besteht darin, daß der Ultrafiltrationskoeffizient gemessen wird. Dieser ändert sich bekanntlich von Dialysator zu Dialysator infolge produktionstechnischer Abweichungen und insbesondere im Laufe der Dialyse, da sich die Membranen verändern bzw. die Poren verstopft werden. Infolgedessen ändert sich auch damit die Ultrafiltrationsmenge, wobei sie regelmäßig im Laufe der Dialyse je Zeiteinheit geringer wird. Daher muß der Ultrafiltrationskoeffizient diskontinuierlich im Laufe der Dialyse bestimmt werden, wobei während dieser Meßdauer der Transmembrandruck TMP konstant gehalten wird. Dies ist bei den Hämofiltrationsgeräten COBE CENTRY 2000 oder Braun UF-Modul der Fall.

Andererseits kann jedoch aber auch die Ultrafiltrationsrate vorgegeben und der sich einstellende mittlere TMP bestimmt werden. Aus der UF-Rate und dem TMP kann wiederum der UF-Koeffizient errechnet und in der Folge der TMP so gesteuert werden, daß die vorgegebene Soll-UF-Rate erzielt wird.

Aus der DE-C-25 48 759 ist ein Dialysegerät bekannt, mit dem die Ultrafiltrationsrate jederzeit während der Blutdialysebehandlung gemessen werden und der Dialysatdruck auf einem Wert gehalten werden kann, der auf einen im Dialysator eingestellten Druck angepaßt ist. Diese Anordnung arbeitet mit jeweils einer Pumpe in der Zulauf- und Ablaufleitung der Dialysierflüssigkeit, wobei diese Pumpen von einem gemeinsamen Motor angetrieben werden und die gleiche Förderkapazität aufweisen.

Abgesehen davon, daß diese Anordnung einen hohen apparativen Aufwand erforderlich macht, ist sie zusätzlich nicht ausreichend genau, da derartige Pumpen einen Fehler in ihrer Förderleistung von etwa 2 % aufweisen. Berücksichtigt man, daß etwa 2 % der den Dialysator durchfliessenden Dialysierflüssigkeitsmengen zusätzlich als Ultrafiltrationsmenge entzogen werden sollen, so ist hieraus bereits ersichtlich, daß mit einer derartigen Anordnung keine genaue Ultrafiltration durchgeführt werden kann und in einigen Fällen sogar der Einsatz einer derartigen Anordnung für den Patienten kritisch sein kann.

Aus der DE-A-32 02 831 ist eine Hämodialysevorrichtung der eingangs erwähnten Art bekannt, bei der für die Zeitdauer der Messung der entnommenen Ultrafiltratmenge der bei einer Single-Pass-Vorrichtung offene Kreislauf geschlossen wird und die entnommene Ultrafiltratmenge in einer Volumenmeßeinrichtung in Abhängigkeit von der Meßdauer bestimmt wird. Die bekannte Dialyseeinrichtung ist insofern nachteilig, als sie einen hohen apparativen Aufwand benötigt, um diesen Meßwert exakt bestimmen zu können. Hieraus resultiert eine Möglichkeit der Fehleranhäufung, so daß es erwünscht ist, eine weniger fehleranfällige Vorrichtung einzusetzen.

Bei der Hämodialysevorrichtung gemäß DE-A-32 02 831 ergibt sich dieser Nachteil zunächst daraus, daß in der Dialysierflüssigkeitsquelle eine weitere Pumpe vorgegeben werden muß, die zwingend notwendig ist, um die Dialysierflüssigkeit in den Dialysierflüssigkeitskreislauf zu fördern. Wird beispielsweise eine Dialysierflüssigkeit falscher Zusammensetzung hergestellt, so enthält ein Dialysegerät üblicher Weise ein Schutzsystem, das dafür sorgt, daß die Dialysierflüssigkeit falscher Zusammensetzung am Dialysator vorbei in eine Bypassleitung in den Ablauf gefördert wird.

Des weiteren weist die bekannte Dialysevorrichtung eine Förderpumpe in Verbindung mit einer Drosseleinrichtung auf, die als Konstantstromeinrichtung ausgebildet sein soll und die in Verbindung mit der stromab des Dialysators angeordneten Saugpumpe einen bestimmten Transmembrandruck einstellen soll. Infolge der üblichen Gangungenauigkeit von etwa 2 %, die an einer derartigen Pumpe festzustellen ist, muß eine derartige Vorrichtung relativ häufig in den Meßbetrieb geschaltet werden, was zur Unterbrechung der Behandlung des Patienten führt. Hinzu kommt, daß mit steigender Zahl der Pumpen, die in einer derartigen Dialysevorrichtung eingesetzt werden, die Zahl der Ventilanordnungen, die Fehleranfälligkeit und die Ungenauigkeit einer derartigen Hämodialysevorrichtung ansteigen, was unerwünscht ist.

Des weiteren zweigt bei der bekannten Hamodialysevorrichtung die Volumenmeßeinrichtung direkt vom Dialysierflüssigkeitskreislauf stromab der Dialysierflüssigkeitsquelle ab, was bei einer längeren Meßdauer den Nachteil hat, daß die Dialysierflüssigkeit abkühlt, mit der Folge, daß der Patient bei der Dialysebehandlung friert.

Aus den DE-A-23 28 593 und 29 26 681 sind Hämodialysevorrichtungen bekannt, bei denen der Dialysierflüssigkeitskreislauf zur Bestimmung des Transmembrandrucks bei einer bestimmten Ultrafiltrationsrate völlig geschlossen wird, wobei an diesen geschlossenen Kreislauf eine exakt volumetrisch arbeitende Pumpe angeschlossen wird. Diese zusätzliche Pumpe erhöht zunächst die Kosten und die Fehleranfälligkeit einer derartigen Vorrichtung. Es wird dabei über eine vorgegebene Pumprate der Transmembrandruck gemessen, mit dem dann wiederum eine weitere als Saugpumpe dienende Pumpe gesteuert wird.

Generell weisen die bekannten Hämodialysevorrichtungen den Nachteil auf, daß sie über ein getrenntes Desinfizierungsprogramm verfügen, um zu verhindern, daß der Patient unbeabsichtigt an die tödlich wirkende Desinfektionsmittellösung angeschlossen wird. Infolgedessen verfügen die bekannten Vorrichtungen über manuell zu betätigende Dialysesteckeinrichtungen, die nach dem Abschalten der Dialysevorrichtung in entsprechende Ausnehmungen der Dialysevorrichtung eingesteckt werden müssen, um die gesamte Vorrichtung zu desinfizieren.

Der Erfindung liegt daher die Aufgabe zugrunde, die Vorrichtung der eingangs erwähnten Art so fortzubilden, daß der apparative Aufwand zur Bestimmung der Ultrafiltrationsrate in Abhängigkeit vom Transmembrandruck möglichst gering ist.

Die Lösung der Aufgabe erfolgt dadurch, daß die von dem Umschaltventil abzweigende Leitung mit der Dialysierflüssigkeitsquelle verbunden ist.

Die erfindungsgemäße Dialysevorrichtung weist zunächst den Vorteil auf, daß eine übliche Single-Pass-Dialysevorrichtung ohne größere Anpassung hierzu umgerüstet werden kann. Dabei muß lediglich die Dialysierflüssigkeitsquelle, insbesondere die Mischkammer der Dialysierflüssigkeitsquelle über eine Verbindungsleitung mit der Dialysierflüssigkeitsableitung stromab der Saugpumpe verbunden werden, um einen Rezirkulationskreislauf zu bilden.

Dieser Rezirkulationskreislauf ist weiterhin mit einer Volumenmeßeinrichtung versehen, die in der Rezirkulationsphase gegenüber der Atmosphäre offen ist. Diese Volumenmeßeinrichtung ist vorteilhafterweise ebenfalls mit der Dialysierflüssigkeitsquelle, insbesondere mit der Mischkammer der Dialysierflüssigkeitsquelle verbunden und bestimmt in Abhängigkeit von der Zeit die dem Blutkreislauf entzogene Ultrafiltrationsmenge.

Gegenüber den bekannten Dialysevorrichtungen weist die erfindungsgemäße Hämodialysevorrichtung insbesondere den Vorteil auf, daß keine weiteren Pumpen gegenüber der üblichen Single-Pass-Vorrichtung mehr notwendig sind. Insofern muß also die übliche Single-Pass-Vorrichtung lediglich mit einem stromab der Saugpumpe angeordneten Umschaltventil, einer Verbindungsleitung zur Dialysierflüssigkeitsquelle und einer Volumenmeßeinrichtung ausgerüstet sein, die ebenfalls mit der Dialysierflüssigkeitsquelle vorteilhafterweise verbunden ist. Sämtliche bekannten Dialysiervorrichtungen weisen jedoch demgegenüber zusätzliche Pumpen, die gegebenenfalls sogar volumengenau arbeiten müssen, und zusätzliche Ventileinrichtungen auf, die die vorstehend beschriebenen Nachteile bei der Bestimmung dieser Ultrafiltrationsrate besitzen. Demgegenüber kann mit der erfindungsgemäßen Vorrichtung mit einem minimalen apparativen Aufwand die Ultrafiltrationsrate in Abhängigkeit vom Transmembrandruck bestimmt werden.

Diese Ultrafiltrationsrate wird als Ist-Wert in die Regeleinrichtung eingegeben, die aufgrund des zu Beginn der Dialyse eingegebenen Soll-Werts, also der zu ultrafiltrierenden Menge während der Dialyse, die Saugpumpe entsprechend regelt. So wird also durch Erhöhung der Drehzahl der Saugpumpe der Transmembrandruck TMP und damit die Ultrafiltrationsrate erhöht, die im wesentlichen dem TMP proportional ist. Andererseits kann natürlich auch die Saugleistung, also die Drehgeschwindigkeit der Saugpumpe, verringert werden, mit der Folge, daß der TMP und somit die Ultrafiltrationsrate sinkt. Üblicherweise wird jedoch infolge der während der Dialyse sich einstellenden Verschlechterung der Ultrafiltrationsrate die Saugleistung der Saugpumpe erhöht, um die vorbestimmte Ultrafiltrationsrate wieder zu erreichen.

Gemäß einer weiteren Ausführungsform kann der Ultrafiltrationskoeffizient aus der UF-Rate und dem TMP ermittelt werden. Hierzu wird der mittlere TMP mit Hilfe von Druckaufnehmern festgestellt, die auf der Blutseite und der Dialysierflüssigkeitsseite angeordnet sind. Der tatsächliche UF-Koeffizient wird dann in der Regeleinrichtung mit dem ursprünglich, d. h. am Beginn der Dialyse gemessenen UF-Koeffizienten verglichen und durch Veränderung des Drucks, d.h. der Drehzahl der Saugpumpe, an diesen Wert angepaßt.

Gemäß einer weiteren Ausführungsform kann die die Dialysierflüssigkeitsquelle mit der Ablaßleitung verbindende Leitung als Rezirkulationsleitung für eine Desinfektionsmittellösung vorgesehen werden. Diese wird in die Ablaßleitung zwischen der Saugpumpe und dem Dialysator mit Hilfe einer Zuführungsleitung eingespeist, die ständig an einen Desinfektionsmittelkonzentratbehälter angeschlossen ist. Diese Zuführungsleitung ist mit Hilfe eines Absperrorgans geschlossen, das nur dann geöffnet wird, wenn das Hämodialysegerät in das Desinfektionsprogramm gebracht wird.

Dieses Verfahren ist entsprechend den Richtlinien für medizinisch-technische Vorrichtungen als sicher anzusehen, da wenigstens zwei Fehler auftreten müssen, um einen gefährlichen Zustand zu erzeugen. So müßte das Gerät unbeabsichtigt in die Rezirkulation gehen, was unschwer gemessen werden kann, da der Nettowasserzufluß stoppt, und es müßte das Desinfektionsmittelventil zugleich geöffnet werden.

Gemäß einer weiteren vorteilhaften Ausführungsform kann die Desinfektionsmittelzuführungsleitung auch stromab der Saugpumpe mit der Dialysierflüssigkeitsableitung verbunden sein, so daß das Desinfektionsmittel im Überdruckbereich zugeführt wird. Würde das oben erwähnte Ventil in der Desinfektionsmittelleitung lecken, so wäre auch hier ein Ansaugen von Desinfektionsmittel ausgeschlossen, da der stromab der Saugpumpe vorliegende Überdruck dies verhindern würde. Um diesen zu überwinden, ist in der Leitung für die Zuführung des Desinfektionsmittelkonzentrats eine separate Pumpe vorgesehen, so daß hierdurch das System weiter sicherer gemacht wird.

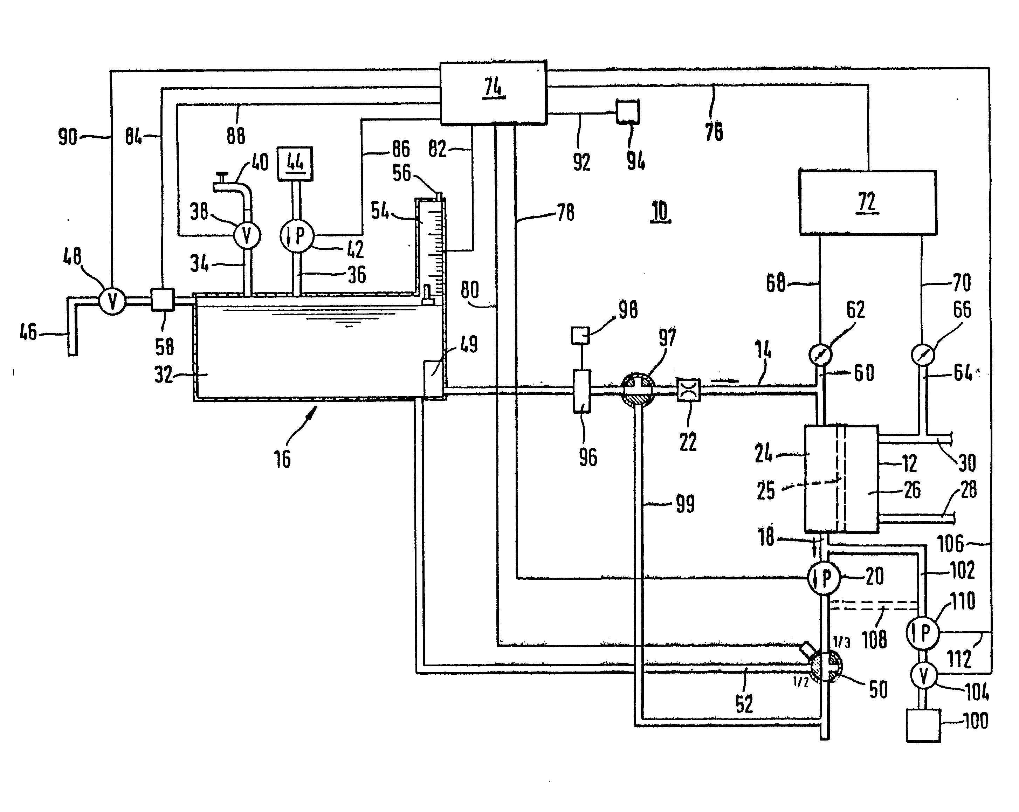

Weitere Merkmale, Einzelheiten und Vorteile der Erfindung sind anhand der nachfolgenden Beschreibung von Ausführungsbeispielen unter Bezugnahme auf die Zeichnung erläutert.

Die Figur zeigt schematisch eine Ausführungsform der erfindungsgemäßen Hämodialysevorrichtung.

Aus der Figur ist mit 10 eine Hämodialysevorrichtung ersichtlich, die aus einem Dialysator 12 besteht, der über eine Zuleitung 14 mit einer Dialysierflüssigkeitsquelle 16 verbunden ist. An den Auslaß des Dialysators 12 ist eine Ablaßleitung 18 angeschlossen, in die eine Saugpumpe 20 eingeschaltet ist.

In die Zuleitung 14 ist weiterhin eine Drossel 22 eingeschaltet, die vorteilhafterweise fest eingestellt ist. Weiterhin ist in der Zuleitung 14 eine nicht gezeigte Vorrichtung zur Entlüftung der Dialysierflüssigkeit vorgesehen.

Die Dialysierflüssigkeitsquelle 16, die Zuleitung 14 und die Ableitung 18 bilden in Verbindung mit der von der Dialysierflüssigkeit durchflossenen Kammer 24 des Dialysators 12 einen Dialysierflüssigkeitsweg. Die andere Kammer 26, die durch die Membran 25 von der Kammer 24 getrennt ist, wird von Blut durchflossen und ist zu diesem Zweck mit einer Zuleitung 28 und einer Ableitung 30 verbunden.

Die Dialysierflüssigkeitsquelle 16 besteht gemäß der in der Figur gezeigten Ausführungsform aus einer Mischkammer 32, in die eine Leitung 34 zur Zuführung von Frischwasser und wenigstens eine Leitung 36 zur Zuführung eines Konzentrats führen. Zu diesem Zweck ist die Leitung 34, in die ein Ventil 38 eingeschaltet ist, mit einem Frischwasseranschluß 40 verbunden, während die Leitung 36, in die eine Konzentratpumpe 42 eingeschaltet ist, mit wenigstens einem Konzentratbehälter 44 verbunden ist.

Weiterhin weist die Mischkammer 32 an ihrer Oberseite eine Abflußleitung 46 auf, durch die überfließende Dialysierflüssigkeit ablaufen kann. In dieser Abflußleitung 46 ist ein Ventil 48 vorgesehen.

Eine derartige Mischkammer 32 in Verbindung mit diesen Zuführungs- und Abführungsleitungen ist bekannt, so daß auf die Beschreibung der Dialysierflüssigkeit selbst verzichtet wird.

Die Mischkammer 32 weist bei ihrem Übergang in die Zuleitung 14 eine Entgasungs- und Fördereinrichtung 49 auf, in der üblicherweise eine Drossel und eine Pumpe vorgesehen sind, wobei letztere die entgaste Dialysierflüssigkeit in die Zuleitung 14 fördert.

Die Abflußleitung 18 weist stromab der Saugpumpe 20 eine Ventilanordnung 50 auf die in der Stellung 1/3 die Ablaßleitung 18 unmittelbar mit dem Abfluß verbindet, während sie in der Stellung 1/2 die Ablaßleitung 18 über eine Leitung 52 mit der Dialysierflüssigkeitsquelle 16, insbesondere mit der Mischkammer 32, verbindet. Vorteilhafterweise mündet die Leitung 52 im Bereich des Bodens der Mischkammer 32.

Die Oberseite der Mischkammer 32 weist gemäß der in der Figur gezeigten Ausführungsform eine Volumenmeßeinrichtung 54 auf, die vorteilhafterweise über eine Entlüftungsöffnung 56 druckausgeglichen ist. Diese Entlüftungsöffnung 56 kann mit einer luftdurchlässigen sterilen Membran verschlossen sein.

Als Volumenmeßeinrichtungen kommen sämtliche bekannten Flüssigkeitsmeßeinrichtungen in Frage, die volumetrisch arbeiten, also ein Füllstandsrohr mit Schwimmeranordnung, Ultraschallpegelmeßvorrichtungen, Lichtschranken, die den Füllstandsmesser überwachen, u.dgl.

Anstelle dieser ersten Volumenmeßeinrichtung 54 kann gemäß einer weiteren Ausführungsform eine zweite Volumenmeßeinrichtung 58 in der Abfluß- oder Überlaufleitung 46 vorgesehen sein. In diesem Fall ist die Volumenmeßeinrichtung 58 vorteilhafterweise als Durchflußmesser oder als Turbine ausgebildet, die die überlaufende Dialysierflüssigkeitsmenge bestimmt, und - wie nachstehend erläutert - ist das Ventil 48 in der Überlaufleitung 46 nicht mehr notwendig und kann somit entfallen.

Gemäß einer weiteren vorteilhaften Ausführungsform ist die Zuführungsleitung 14 über eine Leitung 60 mit einem Druckmeßgerät 62 zur Bestimmung des Drucks in der von der Dialysierflüssigkeit durchflossenen Kammer 24 des Dialysators 12 verbunden, während die Blutleitung 30 über eine Leitung 64 mit einem weiteren Druckmeßgerät 66 verbunden ist. Diese Druckmeßgeräte 62 und 66 sind über elektrische Leitungen 68 und 70 mit einer Druckauswerteeinheit 72 verbunden, mit der der an der Membran 25 anliegende Transmembrandruck TMP durch Bildung der Differenz der von den Druckmeßgeräten 62 und 66 angegebenen Druckwerte ermittelt wird.

Das Druckmeßgerät 62 kann wahlweise auch stromab des Dialysators 12 angebracht sein. Auch ist eine Ausführungsform möglich, bei der sowohl stromab als auch stromauf des Dialysators 12 im Blut und/oder im Dialysierflüssigkeitskreislauf je ein Druckmeßgerät 62, 66 angeordnet ist, um auf diese Weise eine Bestimmung und gegebenenfalls Regelung des mittleren TMP (s. DIN 57750, Teil 1206) zu ermöglichen.

Die Hämodialysevorrichtung 10 weist weiterhin eine Regeleinrichtung 74 auf, die über eine elektrische Leitung 76 mit der Druckauswerteeinheit 72 verbunden ist. Weiterhin ist die Regeleinrichtung 74 über eine elektrische Leitung 78 mit der Saugpumpe 20, eine Leitung 80 mit der Ventilanordnung 50, eine Leitung 82 mit der Volumenmeßeinrichtung 54 bzw. eine Leitung 84 mit der Volumenmeßeinrichtung 58, eine elektrische Leitung 86 mit der Konzentratpumpe 42, eine Leitung 88 mit dem Ventil 38 in der Frischwasserleitung 34 und eine elektrische Leitung 90 mit dem in der Abflußleitung 46 vorgesehenen Ventil 48 verbunden. Vorteilhafterweise ist die Regeleinrichtung 74 über eine weitere elektrische Leitung 92 mit einer Sollwerteingabeeinrichtung 94 verbunden.

In der Regeleinrichtung 74 wird zunächst mit Hilfe der Sollwerteingabeeinrichtung 94 die zu entziehende Ultrafiltratmenge, insbesondere die Ultrafiltrationsrate, eingestellt. Da bei den eingesetzten Dialysatoren regelmäßig der Ultrafiltrationskoeffizient zu Beginn der Dialyse bekannt ist und ggf. ebenfalls über die Sollwerteingabeeinrichtung 94 in die Regeleinrichtung 74 eingegeben werden kann, läßt sich die Dialysevorrichtung 10 ohne Schwierigkeiten durch Einregeln des TMP mit Hilfe der Saugpumpe anfahren, da der Ultrafiltrationsrate ein bestimmter TMP entspricht.

Ist die Hämodialysevorrichtung 10 in Betrieb, so erfolgt die weitere Regelung der Pumpe 20 in regelmäßigen Abständen, d.h. nach etwa 10 - 30 Minuten nach dem nachfolgend beschriebenen Verfahren.

Soll die Ultrafiltrationsrate überprüft werden, so schaltet die Regeleinrichtung 74 zunächst die Ventilanordnung 50 von der 1/3- auf die 1/2-Stellung über die Leitung 80 um. Zugleich wird das Ventil 48 in der Abflußleitung 46 geschlossen, sofern die Volumenmeßeinrichtung 54 verwendet wird. Ist dies nicht der Fall, so wird anstelle der Volumenmeßeinrichtung 54 die Volumenmeßeinrichtung 58 aktiviert.

In der bevorzugten Ausführungsform wird über die Leitung 76 der TMP in die Regeleinrichtung 74 eingegeben, während die gemessenen Volumina in den Volumenmeßeinrichtungen 54 bzw. 58 ebenfalls in Abhängigkeit von der Zeit in die Regeleinrichtung 74 eingegeben werden. Zu Beginn der Bestimmung werden ebenfalls der Frischwasserzufluß durch Schließen des Ventils 38 und die Konzentratzuführung durch Abstellen der Pumpe 42 unterbrochen.

In dem hierdurch gebildeten Zirkulationskreislauf wird das Ultrafiltrat, das durch den Unterdruck in der Unterdruckstrecke zwischen der Drossel 22, dem Dialysator 12 und der Saugpumpe 20 erzeugt wird, als im gebildeten Rezirkulationskreislauf überschüssige Menge entweder durch den Überlauf 46 hinausbefördert oder in die als Steigrohr ausgebildete Volumenmeßeinrichtung 54 gepumpt.

In diesen beiden Volumenmeßeinrichtungen 54 und 58 erfolgt die Bestimmung der Ultrafiltratmenge, deren Wert in Abhängigkeit von der Zeit in der Regeleinrichtung 74 als Ist-Wert mit dem eingegebenen Soll-Wert verglichen wird. Sofern diese Werte nicht übereinstimmen, erfolgt eine Regelung der Saugpumpe 20 mit Hilfe der Regeleinrichtung 74.

In einer weiteren Ausführungsform wird zusätzlich der TMP bei dieser Messung in der Regeleinrichtung 74 gespeichert und der Ultrafiltrationskoeffizient aus dem Quotienten von Ultrafiltrationsrate und TMP bestimmt. Dieser kann in entsprechender Weise mit dem zu Beginn der Dialyse registrierten Ultrafiltrationskoeffizienten verglichen werden, wobei die Abweichung von dem ursprünglichen Wert als Größe zur Regelung des TMP dient.

Nachdem die Ultrafiltrationsrate auf den vorbestimmten Wert eingeregelt ist, wird die Ventilanordnung 50 auf die Stellung 1/3 gebracht, die Ventile 38 und 40 geöffnet und die Konzentratpumpe 42 erneut betätigt. Zugleich wird die Regeleinrichtung 74 von den Meßwertabgebern abgetrennt.

Während der Messung bleibt natürlich der TMP konstant, d.h. die Förderleistung der Saugpumpe 20 wird während der Meßzeit nicht verändert. Sollte dies nicht ausreichen, so wird vorteilhafterweise der Transmembrandruck mit Hilfe der Druckmeßgeräte 62 und 66, der Druckauswerteeinheit 72 und der Regeleinrichtung 74 auf den konstanten Wert eingestellt, wobei die Regeleinrichtung die Regelung eines konstanten TMP an der Pumpe 20 vornimmt.

Zur Ultrafiltrations-Mengenmessung bei isolierter Ultrafiltration kann an den Überlauf 46 ein Meßgefäß angeschlossen werden. Es wird dann während der isolierten Ultrafiltration der Dialysator 12 ständig mit warmer, aber rezirkulierender Dialysierflüssigkeit durchspült und damit ein Auskühlen verhindert. Diese isolierte Ultrafiltration wird üblicherweise ohne Dialysierflüssigkeitsfluß betrieben, um eine Diffusion von Elektrolyten zu vermeiden. Das gleiche Ziel wird mit der Rezirkulation mit der vorstehenden Vorrichtung erreicht.

Durch die Rezirkulation wird - wie vorstehend erwähntein Elektrolytgleichgewicht zwischen Blut und der rezirkulierenden Dialysierflüssigkeit hergestellt, so daß die Konzentration der Elektrolyte in der Dialysierflüssigkeit einen Wert annimmt, bei dem kein Nettoelektrolyttransfer von Blut in die Dialysierflüssigkeit und zurück mehr stattfindet. Aus der Leitfähigkeit der frischen Dialysierflüssigkeit und derjenigen der rezirkulierenden Dialysierflüssigkeit kann dann auf den Elektrolyttransfer bei der Dialyse geschlossen werden, was ein vorteilhafter Nebeneffekt der erfindungsgemäßen Vorrichtung ist. Dies kann beispielsweise mit einem Leitfähigkeitsmesser 96, der in der Zuleitung 14 vorgesehen ist, gemessen und in einem Anzeigegerät 98, das mit dem Leitfähigkeitsmesser 96 verbunden ist, dargestellt werden.

Stromab des Leitfähigkeitsmessers 96 ist in die Zuleitung 14 ein Bypassventil 97 eingeschaltet, von dem eine Bypassleitung 99 abgeht, die mit der Ableitung 18 verbunden ist. Sofern der Leitfähigkeitsmesser eine Dialysierflüssigkeit falscher Zusammensetzung erkennt, wird die Ventilanordnung 97 in den Bypassbetrieb geschaltet, und die Dialysierflüssigkeit falscher Zusammensetzung am Dialysator 12 vorbei unmittelbar in den Abfluß durch die Wirkung der Fördereinrichtung 49 gefördert.

Gemäß einer weiteren vorteilhaften Ausführungsform kann der Hämodialysevorrichtung 10 unmittelbar ein Desinfektionsmittelkonzentrat aus einem Behälter 100 zugeführt werden. Dieser Behälter 100 ist über eine Leitung 102 mit der Ablaßleitung 18 stromauf der Saugpumpe 20 verbunden.

In die Leitung 102 ist ein Ventil 104 eingeschaltet, das sich nur im Desinfektionsprogramm öffnen läßt.

Dieses Desinfektionsprogramm wird mit Hilfe einer Steuervorrichtung durchgeführt, die z. B. mit der Regeleinrichtung 74 vereint sein kann. Sie kann das Ventil 104 über die Leitung 106 und - wie vorstehend erwähnt - das Ventil 50, das Wassereingangsventil 38, gegebenenfalls das Ventil 48 sowie die Pumpe 42 schalten bzw. steuern.

Zur Desinfektion einer vorher mit Wasser freigespülten Maschine wird das Ventil 50 zunächst in die Stellung 1/2 geschaltet, das Wassereingangsventil 38 sowie gegebenenfalls das Ventil 48 geschlossen und die Konzentratpumpe 42 gestoppt. Die Saugpumpe 20 wird so gesteuert, daß an der Ansaugseite ein Unterdruck von typisch 50 - 100 mbar. entsteht. Anschließend wird das Ventil 104 solange geöffnet, bis eine der gewünschten Konzentration entsprechende Menge Desinfektionsmittelkonzentrat angesaugt ist, und anschließend geschlossen. Diese Konzentratmenge wird vermischt und entsprechend der notwendigen Einwirkzeit bei gleichmäßiger Verteilung umgepumpt (rezirkuliert).

Es ist aber auch möglich, nach einer kurzen Umpumpphase, die zur Durchmischung von Konzentrat und Wasser dient, das Gerät abzustellen und eine, im allgemeinen verlängerte Einwirkzeit im Ruhezustand abzuwarten.

Am Ende der Desinfektionsphase wird das Ventil 50 wieder in die Stellung 1/3 geschaltet sowie das Ventil 38 und 48 geöffnet. Anschließend wird die Maschine freigespült.

Die Öffnungszeit des Ventils 104 wird empirisch bestimmt. Bei üblichen Konzentraten, die 1 : 34 verdünnt werden müssen, ergibt sich die notwendige Menge Konzentrat aus dem Füllvolumen des Dialysierflüssigkeitskreislaufs des Hämodialysegerätes 10 dividiert durch 34.

Wird anstelle des Ventils 104 oder zusätzlich hierzu eine Pumpe 110 vorgesehen, so kann diese Menge unschwer durch entsprechende Steuerung dieser Pumpe 110 ausreichend genau gefördert werden.

Anschließend kann wieder dialysiert werden.

Wie bereits vorstehend erwähnt, ist bereits diese Anordnung gegen einen ersten Fehler sicher. Um jedoch die Sicherheit noch weiter zu verbessern, ist die Leitung 102 über die gestrichelt gezeichnete Leitung 108 mit der Ablaßleitung 18 stromab und nicht stromauf der Saugpumpe 20 verbunden, befindet sich somit im Überdruckbereich, so daß auch bei einem unabsichtlich geöffneten Ventil 104 kein Desinfektionsmittelkonzentrat in den Rezirkulationskreislauf gelangen kann. Um diesen Überdruck zu überwinden, ist in der Leitung 102 eine Pumpe 110 vorgesehen, die nur im Desinfektionsprogramm betätigt wird, ansonsten jedoch ebenfalls wie das Ventil 104 die Leitung 102 sperrt. Diese Pumpe ist über eine elektrische Leitung 112 ebenfalls mit der Leitung 106 verbunden und wird somit in Verbindung mit dem Ventil 104 von der Regeleinrichtung 74 betätigt.

Die erfindungsgemäß vorgesehene Rezirkulation ermöglicht folgendes Desinfektionsverfahren:

1. Das Hämodialysegerät 10 wird im Durchfluß (ohne Rezirkulation) mit Wasser freigespült.

2. Anschließend wird es in das Desinfektionsprogramm gebracht, wobei der Rezirkulationskreislauf - wie vorstehend erläutert - aufgebaut wird. Hieran schließt sich die Zuführung des Desinfektionsmittelkonzentrats aus dem Behälter 100 in den Rezirkulationskreislauf an. Es wird eine bestimmte Menge eingeführt, die ausreicht, um die Konzentration des Desinfektionsmittels im ganzen Kreislauf auf das gewünschte Niveau zu bringen.

3. Nach einer Wartezeit, die bis zur nächsten Behandlung dauern kann, wird das Gerät automatisch freigespült. Dabei wird die Rezirkulation aufgehoben und mit Wasser freigespült.

Dieses Verfahren ist deshalb sicherer, weil das Desinfektionsmittel stromab des Dialysators 12 zugeführt wird und erst durch die Rezirkulation den Dialysator 12 wieder erreichen kann. Damit ein gefährlicher Zustand auftreten kann, müssen also zwei Fehler vorliegen:

Das Gerät muß zunächst unbeabsichtigt in die Rezirkulationsstufe übergehen, was unschwer festgestellt werden kann, und es muß das Desinfektionsmittelventil irrtümlich geöffnet werden.

Schließlich sei noch darauf verwiesen, daß als Dialysatquelle 16 die in der DE-A-33 13 421 (=EP-A-0 122 604, veröffentlicht am 24.10.84) erläuterte Einrichtung zur Erzeugung der Dialysierflüssigkeit eingesetzt werden kann, auf deren Offenbarung ausdrücklich Bezug genommen wird. Bei dieser Einrichtung ist die Volumenmeßeinrichtung 54 vorteilhafterweise in dem Heizabschnitt vorgesehen, in den ebenfalls die Leitung 52 mündet. Somit stellt der Heizabschnitt, in den ebenfalls die übrigen Zuführungsleitungen münden, die Mischkammer 32 gemäß der Figur dar.

**Patentansprüche**

1. Hämodialysevorrichtung (10) mit einem Dialysator (12), der zwei durch eine Membran (25) getrennte Kammern (24, 26) aufweist, von denen die eine (24) in einen Dialysierflüssigkeitsweg und die andere (26) in einen Blutweg geschaltet ist, mit einer Dialysierflüssigkeitsquelle (16), mit einer im Dialysierflüssigkeitsweg angeordneten, den Transmembrandruck regelnden Saugpumpe (20), mit einer stromauf des Dialysators im Dialysierflüssigkeitsweg angeordneten Drosseleinrichtung (22), mit einem stromab der Saugpumpe in den Dialysierflüssigkeitsweg eingeschalteten Umschaltventil (50), mit einer von dem Umschaltventil abgehenden Leitung (52), die zum Dialysierflüssigkeitsweg stromauf des Dialysators zurückgeführt ist, mit einer mit dem Dialysierflüssigkeitsweg verbundenen Volumenmeßeinrichtung (54, 58) zur Messung des Volumens der ultrafiltrierten Flüssigkeitsmenge, mit einer Einrichtung (62, 66, 72) zur Bestimmung des Transmembrandrucks im Dialysator und mit einer Regeleinrichtung (74), die mit der Einrichtung zur Bestimmung des Transmembrandrucks verbunden ist und die Saugpumpe (20) regelt, dadurch gekennzeichnet, daß die von dem Umschaltventil (50) abzweigende Leitung (52) mit der Dialysierflüssigkeitsquelle (16) verbunden ist.

2. Hämodialysevorrichtung nach Anspruch 1, dadurch gekennzeichnet, daß die Dialysierflüssigkeitsquelle (16) eine Mischkammer (32) aufweist, an die die Volumenmeßeinrichtung (54, 58) angeschlossen ist.

3. Hämodialysevorrichtung nach Anspruch 2, dadurch gekennzeichnet, daß die Volumenmeßeinrichtung (54) ein Steigrohr ist und über eine Leitung (82) mit der Regeleinrichtung (74) zur Bestimmung der Ultrafiltrationsrate verbunden ist.

4. Hämodialysevorrichtung nach Anspruch 2, dadurch gekennzeichnet, daß die Volumenmeßeinrichtung (58) ein Durchflußmesser oder ein Mengenzähler ist, und über eine Leitung (84) mit der Regeleinrichtung (74) zur Bestimmung der Ultrafiltrationsrate verbunden ist.

5. Hämodialysevorrichtung nach Anspruch 2, dadurch gekennzeichnet, daß die Mischkammer (32) über eine Leitung (36), in die eine Konzentratpumpe(42) eingeschaltet ist, mit einem Konzentratbehälter (44) und über eine Leitung (34), in die ein Ventil (38) eingeschaltet ist, mit einem Frischwasseranschluß (40) verbunden ist und daß die Konzentratpumpe (42) und das Ventil (38) über Leitungen (86, 88) mit der Regeleinrichtung (74) verbunden sind und von dieser in der Meßphase gesperrt werden.

6. Hämodialysevorrichtung nach Anspruch 2, dadurch gekennzeichnet, daß die Mischkammer (32) eine Überlaufleitung (46) aufweist, in die ein Ventil (48) eingeschaltet ist, das über eine Leitung (90) mit der Regeleinrichtung (74) verbunden ist und von diesem in der Rezirkulationsphase gesperrt wird.

7. Hämodialysevorrichtung nach Anspruch 1, dadurch gekennzeichnet, daß die Regeleinrichtung (74) über eine Leitung (80) mit dem Umschaltventil (50), über eine Leitung (76) mit einer Transmembrandruckauswerteinheit, (72), über eine Leitung (92) mit einer Sollwerteingabeeinrichtung (94) und über eine Leitung (78) mit der Saugpumpe (20) verbunden ist.

**Claims**

1. A hemodialysis device (10) with a dialyzer (12) which has two chambers (24, 26) separated by a membrane (25), one (24) of which is switched into a dialyzing liquid path and the other (26) into a blood path, with a dialyzing liquid source (16), with a suction pump (20) arranged in the dialyzing liquid path and controlling the transmembrane pressure, with a restrictor apparatus (22) arranged upstream of the dialyzer in the dialyzing liquid path, with a change-over valve (50) interposed downstream of the suction pump in the dialyzing liquid path, with a line (52) going off from the change-over valve and returning to the dialyzing liquid path upstream of the dialyzer, with a volume-measuring apparatus (54, 58) connected to the dialyzing liquid path for measuring the volume of the ultrafiltered liquid quantity, with an apparatus (62, 66, 72) for determination of the transmembrane pressure in the dialyzer and with a control apparatus (74) which is connected to the device for determination of the transmembrane pressure and controls the suction pump, characterized in that the line (52) branching off from the change-over valve (50) is connected to the dialyzing liquid source (16).

2. The hemodialysis device according to claim 1, characterized in that the dialysis liquid source (16) has a mixing chamber (32) to which the volume-measuring apparatus (54, 58) is connected.

3. The hemodialysis device according to claim 2, characterized in that the volume-measuring apparatus (54) is a riser and is connected via a line (82) to the control apparatus (74) for determination of the ultrafiltration rate.

4. The hemodialysis device according to claim 2, characterized in that the volume-measuring apparatus (58) is a flow meter or a quantity counter and is connected via a line (84) to the control apparatus (74) for determination of the ultrafiltration rate.

5. The hemodialysis device according to claim 2, characterized in that the mixing chamber (32) is connected to a concentrate container (44) via a line (36) in which a concentrate pump (42) is interposed and to a fresh water connection (40) via a line (34) in which a valve (38) is interposed, and that the concentrate pump (42) and the valvle (35) are connected via lines (86, 88) to the control apparatus (74) and are blocked from the latter in the measuring phase.

6. The hemodialysis device according to claim 2, characterized in that the mixing chamber (32) has an overflow line (46) in which a valve (45) is interposed which is connected via a line (90) to the control apparatus (74) and is blocked from the latter in the recirculation phase.

7. The hemodialysis device according to claim 1, characterized in that the control apparatus (74) is connected via a line (80) to the change-over valve (50), via a line (76) to a transmembrane-pressure-evaluation-unit (72), via a line (92) to a set value input apparatus (94) and via a line (78) to the suction pump (20).

**Revendications**

1. Dispositif d'hémodialyse (10) comprenant un dialyseur (2) qui comporte deux chambres (24, 26) séparées par une membrane (25), l'une des chambres (24) étant raccordée à un parcours à liquide de dialyse et l'autre à un parcours à sang, comprenant une source de liquide de dialyse (16), une pompe aspirante (20) montée dans le parcours de liquide de dialyse et régulant la pression de la transmembrane, un dispositif d'étranglement (22) monté en amont du dialyseur dans le parcours à liquide de dialyse, une vanne inverseuse (50) montée en aval de la pompe aspirante dans le parcours à liquide de dialyse, une conduite (52) partant de la vanne inverseuse, qui revient au parcours de liquide de dialyse en amont du dialyseur, un dispositif de mesure de volume (54, 58) relié au parcours de liquide de dialyse et destiné à la mesure du volume de la quantité de liquide soumis à l'ultrafiltration, un dispositif (62, 66, 72) pour déterminer la pression de la transmembrane dans le dialyseur et un dispositif de régulation (74) qui est relié au dispositif de détermination de la pression de la transmembrane et régule la pompe aspirante (20), caractérisé en ce que la conduite (52) qui est en dérivation de la vanne inverseuse (50) est reliée à la source de liquide de dialyse (16).

2. Dispositif d'hémodialyse selon la revendication 1, caractérisé en ce que la source de liquide de dialyse (16) comprend une chambre mélangeuse (32) à laquelle est raccordé le dispositif de mesure de volume (54, 58).

3. Dispositif d'hémodialyse selon la revendication 2, caractérisé en ce que le dispositif de mesure de volume (54) est constitué par une colonne montante et est relié par une conduite (82) au dispositif de régulation (74) pour déterminer le taux d'ultrafiltration.

4. Dispositif d'hémodialyse selon la revendication 2, caractérisé en ce que le dispositif de mesure de volume (58) est constitué par un débitmètre ou par un totalisateur de quantités, et est relié par une conduite (84) au dispositif de régulation (74) pour déterminer le taux d'ultrafiltration.

5. Dispositif d'hémodialyse selon la revendication 2, caractérisé en ce que la chambre mélangeuse (32) est reliée par une conduite (36) dans laquelle est montée une pompe à concentrat (42), comprenant un récipient à concentrat (54), et par une conduite (34) dans laquelle est montée une vanne (38) à un raccord à eau fraîche, et en ce que la pompe à concentrat (42) et la vanne (38) sont reliées par des conduites (86, 88) au dispositif de régulation (74) et sont fermées par ce dernier pendant la phase de mesure.

6. Dispositif d'hémodialyse selon la revendication 2, caractérisé en ce que la chambre mélangeuse (32) comprend une conduite de trop-plein (46) dans laquelle est montée une vanne (48) reliée par une conduite (90) au dispositif de régulation (74) et est fermée par ce dernier pendant la phase de recirculation.

7. Dispositif d'hémodialyse selon la revendication 1, caractérisé en ce que le dispositif de régulation (74) est relié par une conduite (80) à la vanne inverseuse (50), par une conduite (76) à l'unité d'évaluation de pression de la transmembrane (72), par une conduite (92) à un dispositif d'entrée de valeur de consigne (94) et par une conduite (78) à la pompe aspirante (20).

0 167 745